(19) Europäisches Patentamt
European Patent Office
Office européen des brevets

(11) **EP 4 268 776 B1**

(12) **EUROPEAN PATENT SPECIFICATION**

(45) Date of publication and mention
of the grant of the patent:
**11.09.2024  Bulletin 2024/37**

(21) Application number: **22170725.0**

(22) Date of filing: **29.04.2022**

(51) International Patent Classification (IPC):
**A61F 13/15** *(2006.01)*      **A61F 13/505** *(2006.01)*
**A61F 13/66** *(2006.01)*      **A61F 13/49** *(2006.01)*

(52) Cooperative Patent Classification (CPC):
**A61F 13/505; A61F 13/15268; A61F 13/15747;
A61F 13/49003; A61F 13/66**

(54) **RE-USABLE SHELL AND FOLDED DISPOSABLE INSERT**

WIEDERVERWENDBARE SCHALE UND GEFALTETER WEGWERFEINSATZ

COQUE RÉUTILISABLE ET INSERT JETABLE PLIÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**01.11.2023  Bulletin 2023/44**

(73) Proprietors:
• **Ontex BV
9255 Buggenhout (BE)**
• **Ontex Group NV
9320 Erembodegem (BE)**

(72) Inventors:
• **IDELSON, Alissa
53359 Rheinbach (DE)**
• **WEBER, Ainas
53474 Bad Neuenahr-Ahrweiler (DE)**

(74) Representative: **Macchetta, Andrea
Ontex BV
Korte Keppestraat 21
9320 Erembodegem-Aalst (BE)**

(56) References cited:
**US-A1- 2014 257 231     US-A1- 2021 361 498**

EP 4 268 776 B1

## Description

## TECHNICAL FIELD

The disclosure relates to absorbent articles such as disposable absorbent articles, preferably selected from the group consisting of diapers (whether for baby or adults), pants (whether for baby or adults), briefs, and combinations thereof generally of the hybrid type i.e. a re-usable and/or washable outer shell and a disposable (generally single-use) absorbent insert cooperable with said outer shell.

## BACKGROUND

Hybrid-type articles, comprising a re-usable outer shell and disposable absorbent insert, are becoming more and more sought after in the market in view of their sustainable benefits and reduced waste production.

Different designs are available for such products such as described in EP 3 842 017 A1 wherein the outer shell is generally a pant-type component that may comprise elastic components therein and the disposable absorbent insert comprises liquid permeable topsheet, liquid impermeable backsheet and absorbent core sandwiched therebetween and is generally re-fastenably joinable to a crotch region of the outer shell.

Other examples are described in EP 3 659 563 A1 where the disposable insert is re-fastenably joined to the outer shell and the outer shell is in the form of a re-fastenable diaper pant; and EP 3 895 673 A1 wherein the disposable inserts are biodegradable and/or compostable. Further absorbent inserts are known from US2014/257231A1 and US2021/361498A1.

Thus, although there have already been significant advancements in this field, there still remains a need to further improve hybrid-type articles so as to provide improved convenience of use such as ease of assembly by the user whilst keeping the design as simple as possible to permit a greater use of environmentally-friendly materials to improve the overall sustainability of the article.

## SUMMARY

In a first aspect, the disclosure relates to a disposable absorbent insert comprising: a liquid permeable topsheet; a liquid impermeable backsheet; and an absorbent core comprising absorbent material therein and being sandwiched between said topsheet and backsheet; wherein the insert comprises a perimeter formed by first and second transverse edges and first and second longitudinal edges connecting the first and second transverse edges; a longitudinal centerline extending substantially parallel to said first and second longitudinal edges and interposed therebetween such to divide said insert into a first longitudinal half between said longitudinal centerline and said first longitudinal edge and a second longitudinal half between said longitudinal centerline and said second longitudinal edge; and a transverse centerline extending substantially parallel to said first and second transverse edges and interposed therebetween such to divide said insert into a first transverse half between said transverse centerline and said first transverse edge and a second transverse half between said transverse centerline and said second transverse edge; wherein the insert comprises longitudinally extending folds proximal to each said first and second longitudinal edges wherein said folds comprise a contact surface where a first portion of the backsheet is in contact with a second portion of the backsheet and the contact surface is a garment-facing surface of said backsheet.

In a second aspect, the disclosure relates to absorbent assembly comprising a disposable absorbent insert and a re-usable outer shell.

In a further aspect, the disclosure relates to a kit of parts comprising a plurality of disposable absorbent inserts and one or more re-usable outer shells.

In a further aspect, the disclosure relates to a method for the manufacture of disposable absorbent inserts, said method comprising the steps of: (i) providing a first web of material, preferably a liquid permeable nonwoven; (ii) depositing absorbent material onto said first web; (iii) providing a second web of material, preferably a liquid permeable nonwoven, and applying said second web of material over the deposited absorbent material, or folding said first web to enclose the deposited absorbent material therein; (iv) joining the first and second webs together, or the folded first web, at the one or more attachment areas to form an absorbent core comprising absorbent material enclosed by said web(s); (v) optionally applying an acquisition distribution layer; (vi) sandwiching the absorbent core, and preferably the acquisition distribution layer, between a liquid permeable layer and a substantially liquid impermeable layer oppositely disposed thereto, preferably with the acquisition distribution layer being in contact with the liquid permeable layer, to form a disposable absorbent insert; wherein after step (vi) a first folding step is applied wherein first and second longitudinal edges of said insert are tucked-in by folding said edges about a folding axis running parallel to a machine direction such that longitudinally extending folds proximal to each said first and second longitudinal edges are formed wherein said folds comprise a contact surface where a first portion of the substantially liquid impermeable layer forming a backsheet is in contact with a second portion of said backsheet and wherein said contact surface is a garment-facing surface of said backsheet.

## BRIEF DESCRIPTION OF THE FIGURES

Fig. 1 Is an illustration, top planar view (topsheet-side), of an insert in an un-folded state according to an embodiment herein.

**Fig. 2** Is an illustration, top planar view (topsheet-side), of an insert in a folded state according to an embodiment herein.

**Fig. 3** Is an illustration, top planar view (topsheet-side), of an insert in a folded state according to an embodiment herein.

**Fig. 4** Is an illustration, top planar view (topsheet-side), of an insert in a folded state according to an embodiment herein.

**Fig. 5** Is an illustration, cross-section, of an insert in a folded state according to an embodiment herein.

**Fig. 6** Is an illustration, top planar view (body-facing side), of a re-usable outer shell according to an embodiment herein.

**Fig. 7** Illustrates an exemplary process for the production of high-AUL Bio-SAP

## DETAILED DESCRIPTION

Unless otherwise defined, all terms used in disclosing characteristics of the disclosure, including technical and scientific terms, have the meaning as commonly understood by one of ordinary skill in the art to which this disclosure belongs. By means of further guidance, term definitions are included to better appreciate the teaching of the present disclosure.

As used herein, the following terms have the following meanings:
"A", "an", and "the" as used herein refers to both singular and plural referents unless the context clearly dictates otherwise. By way of example, "a compartment" refers to one or more than one compartment.

"About" or "substantially" as used herein referring to a measurable value such as a parameter, an amount, a temporal duration, and the like, is meant to encompass variations of +/-20% or less, preferably +/-10% or less, more preferably +/-5% or less, even more preferably +/-1 % or less, and still more preferably +/-0.1 % or less of and from the specified value, in so far such variations are appropriate to perform in the disclosed disclosure. However, it is to be understood that the value to which the modifier "about" or "substantially" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The expression "% by weight" or "%wt" (weight percent), here and throughout the description unless otherwise defined, refers to the relative weight of the respective component based on the overall weight of the formulation.

The use of the term "layer" can refer, but is not limited, to any type of substrate, such as a woven web, nonwoven web, films, laminates, composites, elastomeric materials, absorbent materials (such as SAP and cellulose fibers/fluff mixtures), or the like. A layer can be liquid and air permeable, permeable to air but impermeable to liquids, impermeable both to air and liquid, or the like. When used in the singular, it can have the dual meaning of a single element or a plurality of elements, such as a laminate or stacked plural sub-layers forming a common layer.

The terms "nonwoven", "nonwoven layer" or "nonwoven web" are used interchangeably to mean an engineered fibrous assembly, primarily planar, which has been given a designed level of structural integrity by physical and/or chemical means, excluding weaving, knitting or papermaking (ISO 9092:2019 definition). The directionally or randomly orientated fibers, are bonded by friction, and/or cohesion and/or adhesion. The fibers may be of natural or synthetic origin and may be staple or continuous filaments or be formed in situ. Commercially available fibers have diameters ranging from less than about 0.001 mm to more than about 0.2 mm and they come in several different forms such as short fibers (known as staple, or chopped), continuous single fibers (filaments or monofilaments), untwisted bundles of continuous filaments (tow), and twisted bundles of continuous filaments (yam). Nonwoven webs can be formed by many processes such as meltblowing, spunbonding, solvent spinning, electrospinning, carding and airlaying. The basis weight of nonwoven webs is usually expressed in grams per square meter (g/m2 or gsm).

The term "disposable" refers to absorbent articles and/or inserts that generally are not intended to be laundered or otherwise restored or reused as absorbent articles, i.e., they are intended to be discarded after a single use and, preferably, to be recycled, composted or otherwise disposed of in an environmentally compatible manner.

The term "disposed" is used to mean that an element(s) is formed (joined and positioned) in a particular place or position as a unitary structure with other elements or as a separate element joined to another element.

The terms "interior" and "exterior" refer respectively to the location of an element that is intended to be placed against or toward the body of a wearer when an absorbent article is worn and the location of an element that is intended to be placed against or toward any clothing that is worn over the absorbent article. Synonyms for "interior" and "exterior" include, respectively, "inner" and "outer", as well as "inside" and "outside", or "body-facing" and "garment-facing". Also, when the absorbent article is oriented such that its interior faces upward, e.g., when it is laid out in preparation for setting the wearer on top of it, synonyms include "upper" and "lower" and "top" and "bottom", respectively.

The term "joined" refers to configurations whereby an element is directly secured to another element by attaching the element directly to the other element, and configurations whereby an element is indirectly secured to another element by attaching the element to intermediate member(s) which in turn are attached to the other element.

The term "lateral" or "transverse" refers to a direction running at a 90 degree angle to the longitudinal direction and when combined with the term "substantially" includes directions within $\pm 45°$ of the lateral direction.

The term "longitudinal" refers to a direction running parallel to the maximum linear dimension of the article and when combined with the term "substantially" includes directions within $\pm 45°$ of the longitudinal direction.

"Plant-based fibers", as used herein, includes both harvested fibers and synthetic fibers that comprise bio-based content. Harvested plant-based fibers include cellulosic matter, such as wood pulp; seed hairs, such as cotton; stem (or bast) fibers, such as flax and hemp; leaf fibers, such as sisal; and husk fibers, such as coconut. Assessment of the renewably based carbon in a material can be performed through standard test methods. Using radiocarbon and isotope ratio mass spectrometry analysis, the bio-based content of materials can be determined. ASTM International has established a standard method for assessing the bio-based content of materials. The ASTM method is designated ASTM D6866-10. The application of ASTM D6866-10 to derive a bio-based content and the analysis is performed by deriving a ratio of the amount of organic radiocarbon (14C) in an unknown sample to that of a modern reference standard. The ratio is reported as a percentage with the units "pMC" (percent modern carbon). The modern reference standard used in radiocarbon dating is a NIST (National Institute of Standards and Technology) standard with a known radiocarbon content equivalent approximately to the year AD 1950. AD 1950 was chosen since it represented a time prior to thermo-nuclear weapons testing which introduced large amounts of excess radiocarbon into the atmosphere with each explosion (termed "bomb carbon"). The AD 1950 reference represents 100 pMC.

"Bio-based content" refers to the amount of carbon from a renewable resource in a material as a percent of the mass of the total organic carbon in the material, as determined by ASTM D6866- 10, method B. In order to apply the methodology of ASTM D6866-10 to determine the bio-based content of any absorbent article or component thereof, a sample can be ground into particulates less than about 20 mesh using known grinding methods (e.g., WILEY mill), and a representative sample of suitable mass taken from the randomly mixed particles. Alternatively, if the sample is merely a layer of material, then the layer itself can be analyzed without the need for a pre-grinding step. Note that any carbon from inorganic sources such as calcium carbonate is not included in determining the bio-based content of the material.

Embodiments of the articles according to the disclosure will now be described. It is understood that technical features described in one or more embodiments maybe combined with one or more other embodiments without departing from the intention of the disclosure and without generalization therefrom, especially when such combinations are explicitly or implicitly inferred.

THE OUTER SHELL

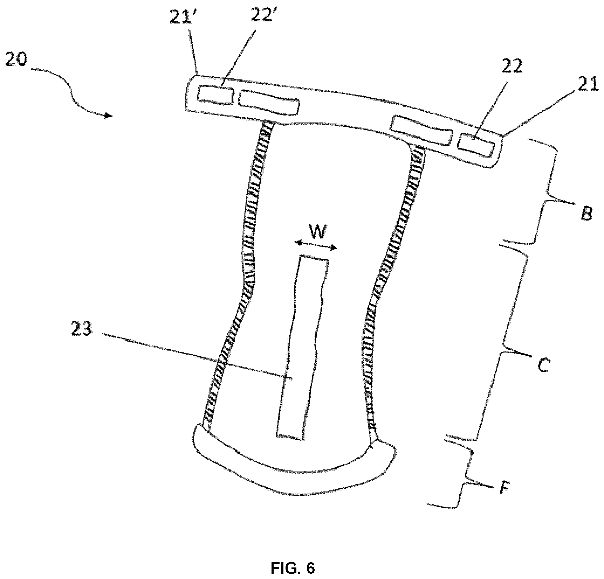

As exemplified in Fig. 6, re-usable outer shells (20) herein preferably comprise: a front (F) and back (B) waist region having uppermost and lowermost edges; and a crotch region (C) interposed between the front and back waist region (F, B), wherein the uppermost edges of the waist region form a waist opening and the lowermost edges of the waist region together with lateral extremities of the crotch region form two oppositely disposed leg openings; and wherein at least the body-facing surface of the crotch region (C) is adapted for receiving an absorbent insert (1) as described herein.

In an embodiment, at least a portion of said crotch region (C) corresponding with a wetness indicator (when present) of the insert (1) is translucent such that said wetness indicator is viewable from a garment facing side of said re-usable outer shell (20) or comprises an optical sensor (OS) adapted to automatically detect a colour change of said wetness indicator and send a corresponding signal to an application device. Advantageously this not only allows to provide effective saturation indication when using a hybrid-article but further allows for at the same time providing indicia to correctly position the insert in the right front/back relationship that may be particularly useful when designing inserts that have a non-uniform absorption profile (e.g. varying basis weight of absorbent material for optimised absorption and limited over-specked or wasted material which then require the correct front/back orientation on placement). In the latter case the insert may be correctly placed when the wetness indicator is fully/entirely viewable through the outer shell (when viewed on the garment side thereof). This may be verified either directly, i.e. visually, by a caregiver viewing the wetness indicator indicia or indirectly, by automatic identification by the sensor that may then provide a warning via an application device to a caregiver accordingly.

The re-usable outer shell (20) may comprise refastenable or permanent side seams joining the front and back waist regions, preferably in the form of a pant; or the back waist region (B) comprises transversely extending side panels (21, 21') each comprising a fastener (22, 22') for coupling to a landing zone positioned at a garment facing side of the front waist region (F), preferably in the form of a diaper.

In an embodiment the crotch region (C) of the re-usable outer shell (20) comprises a window (23) for viewing said wetness indicator from a garment-facing side of said shell (20). The crotch region (C) is generally adapted for receiving an absorbent insert (1) as described herein and wherein at least a portion and/or area of said crotch region comprises said window (23) that substantially corresponds with the position of the wetness indicator, when the insert (1) is joined to the shell (20). Advantageously this allows for visual inspection of saturation and/or correct positioning directly by a subject on use.

The window herein may be in the form of a cut-out or opening, or may comprise a translucent and/or transparent material such as a film or other suitable synthetic covering.

THE INSERT

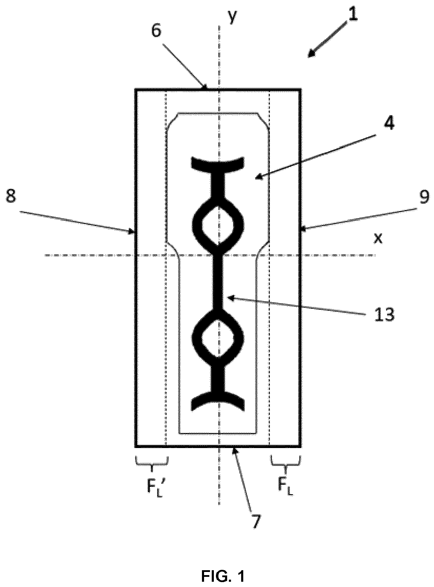

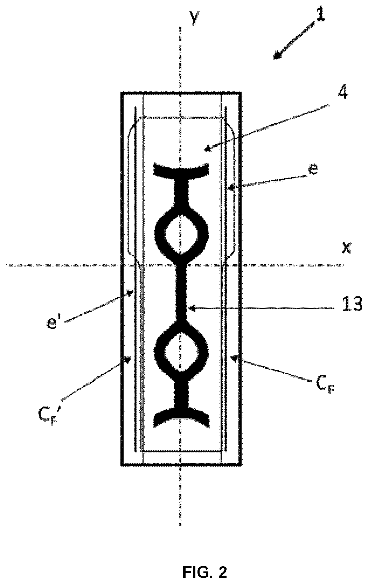

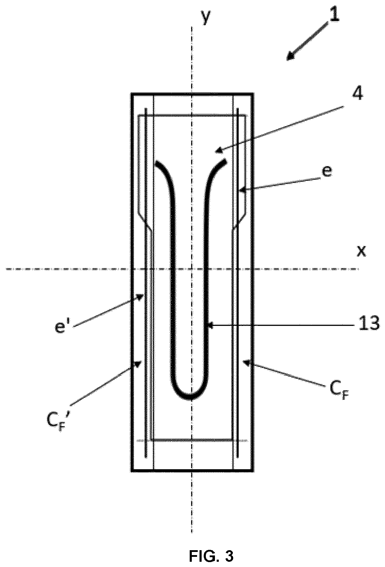

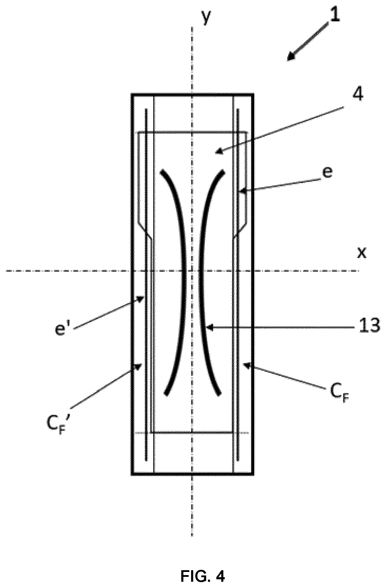

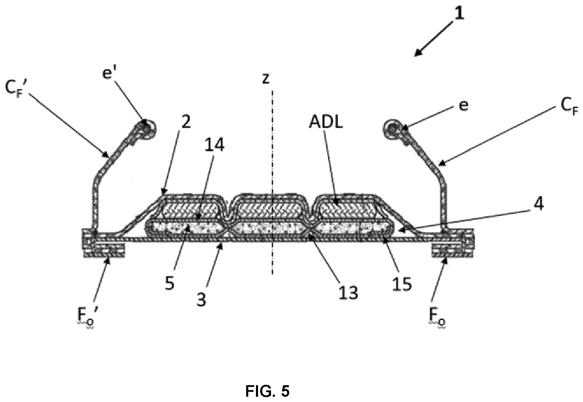

As exemplified in Figs. 1-5, inserts (1) herein typically comprise a liquid permeable topsheet (2), a liquid impermeable backsheet (3); and an absorbent core (4) comprising absorbent material (5) therein and being sandwiched between said topsheet (2) and backsheet (3).

The inserts (1) herein comprise longitudinally extending folds ($F_o$, $F_o$') proximal to each of first and second longitudinal edges (8, 9) wherein said folds ($F_o$, $F_o$') comprise a contact surface where a first portion of the backsheet (3) is in contact with a second portion of the backsheet (3) and wherein said contact surface is a garment-facing surface of said backsheet (3). Advantageously this allows to reduce the footprint of the insert as well as importantly create a stiffening structure at the longitudinal edges for ease of handling and coupling to a re-usable outer shell, this without the need of added stiffening structures to be incorporated.

Inserts herein may further comprise an acquisition distribution layer (ADL) positioned between the topsheet and the absorbent core, preferably between the topsheet and the top core wrap layer. The acquisition distribution layer may have a basis weight of from 15 gsm to 55 gsm, preferably from 18 gsm to 50 gsm, even more preferably from 19 gsm to 45 gsm. Preferably the acquisition distribution layer is selected from a carded airthrough bonded nonwoven, a carded thermobonded calendered nonwoven, a spunbond nonwoven, and combinations thereof.

Generally, the insert comprises a perimeter formed by first and second transverse edges (6, 7) and first and second longitudinal edges (8, 9) connecting the first and second transverse edges (6, 7); a longitudinal centerline extending (y) substantially parallel to said first and second longitudinal edges (8, 9) and interposed therebetween such to divide said insert (1) into a first longitudinal half between said longitudinal centerline (y) and said first longitudinal edge (8) and a second longitudinal half between said longitudinal centerline (y) and said second longitudinal edge (9); and a transverse centerline (x) extending substantially parallel to said first and second transverse edges (6, 7) and interposed therebetween such to divide said insert (1) into a first transverse half between said transverse centerline (x) and said first transverse edge (6) and a second transverse half between said transverse centerline (x) and said second transverse edge (7).

Preferably, the insert comprises oppositely disposed longitudinally extending flanges ($F_L$, $F_L$') along the first and second longitudinal edges (8, 9) and wherein said flanges ($F_L$, $F_L$') are folded to form the longitudinally extending folds ($F_o$, $F_o$'), preferably wherein the longitudinally extending flanges ($F_L$, $F_L$') are substantially free of absorbent material. Preferably, the longitudinally extending flanges ($F_L$, $F_L$') comprise the topsheet (2) and the backsheet (3) being directly or indirectly joined to each other (when indirectly joined it preferably means that the acquisition distribution layer and/or portion of cuffs as described herein are interposed between the topsheet and backsheet when the layers are joined together). Advantageously this allows to form stiffening lateral structures whilst minimising material usage.

In an embodiment, the longitudinally extending folds ($F_o$, $F_o$') comprise adhesive joining the longitudinally extending flanges ($F_L$, $F_L$') to a garment-facing surface of the backsheet (3). The adhesive may be continuously or discontinuously disposed along a longitudinal axis extending substantially parallel to the longitudinal centreline (y). Advantageously this allows for further resistance to unfolding which may bring about further benefits especially in terms of mechanical resistance to bending.

Inserts (1) herein may further comprise a wetness indicator that is viewable from a garment-facing side of the backsheet (3) and the insert (1) is joinable to a re-usable outer shell (20) preferably such that the wetness indicator is viewable from a garment-facing side of the re-usable outer shell (20).

Inserts herein preferably comprise a pair of barrier cuffs extending along the first and second longitudinal edges (8, 9) and arranged to provide lateral barriers preventing exudate leakage. Typically, the barrier cuffs comprising a hydrophobic nonwoven and one or more elastics at an apex position thereof such to form standing gathers when the insert is extended for placement within the re-usable outer shell.

Preferably, inserts (1) herein comprise at least a pair of lateral cuffs ($C_F$, $C_F$') wherein at least one of said cuffs is positioned along at least a portion of the first longitudinal edge (8) and at least one of said cuffs is positioned along at least a portion of the second longitudinal edge (9), preferably wherein the cuffs comprise one or more elastics (e, e') proximal to an apex thereof such that a tension thereof causes the cuffs to project upwards away from the topsheet (2). Preferably, the cuffs are positioned substantially inboard of longitudinally extending flanges ($F_L$, $F_L$') but may also be comprised in at least a portion thereof. Advantageously the cuffs not only provide barriers to liquid as generally known in the art but surprisingly synergistically cooperate with the lateral folds to provide a stiffened structure that aids insert location and attachment to a re-usable outer shell. Moreover, better cup formation has been observed when joined to the outer shell and such aiding in better fit and core utilisation when soiling.

Generally, inserts herein are free of transversely extending side panels such as elastic side panels or elastic ears that are rather commonly found in diapers.

Topsheets (2) for use in inserts herein are preferably selected from nonwovens comprising plant-based fibers wherein said plant-based fibers comprise, preferably consist of, harvested fibers other than wood pulp, such that said topsheet (2) has a bio-based content of from about 10% to about 100%, preferably from about 15% to about 100%, even more preferably from about 20% to about 100%, using ASTM D6866-10, method B, and optionally wherein said nonwoven further comprises synthetic fibers generally in an amount to provide added structural integrity such as from 10% to 60% by weight of said nonwoven.

Backsheets for use herein may be breathable and/or comprise a film, preferably polyethylene (PE) based or bio-PE based, and optionally a nonwoven layer with the nonwoven layer being positioned on the outermost surface at a garment facing side of said film. When present, the nonwoven layer may be similar in composition to that described above for the topsheet but may differ in physical parameters such as basis weight, embossments, fiber bonding mechanism and the like.

Preferably, at least 85%, preferably at least 90%wt, more preferably at least 95%wt, by total weight of the disposable absorbent inserts herein (typically all measurements taken in dry/un-used state i.e. on non-soiled inserts) are composed of materials having a bio-based content of from about 10% to about 100%, preferably from about 15% to about 100%, even more preferably from about 20% to about 100%, using ASTM D6866-10, method B. Advantageously the simple insert designs herein enable the use of high bio-based content materials that would otherwise not be possible without compromising at least one of ease of handling, core utilisation performance, acquisition speed, and liquid retention, as described herein.

In an embodiment, each component or layer forming the disposable absorbent inserts herein has a bio-based content of from about 10% to about 100%, preferably from about 15% to about 100%, even more preferably from about 20% to about 100%, using ASTM D6866-10, method B.

The inserts herein may be substantially rectangular in shape but may equally be shaped such as hourglass and/or substantially T-shaped. Other anatomical shapes are further contemplated herein and suitable in the present disclosure.

In an embodiment, the absorbent material comprises, preferably consists of, superabsorbent particles and/or cellulose fibers.

The absorbent cores herein may comprise at least 60%wt of superabsorbent particles, in particular at least 70%wt, preferably at least 80%wt, preferably at least 90%wt, by total weight of the core.

Preferably, the absorbent material comprises superabsorbent polymer particles preferably selected from Low-AUL Bio-SAP and High-AUL Bio-SAP as described herein. Advantageously this allows to increase the bio-based content of the disposable absorbent insert.

In an embodiment, the superabsorbent particles comprise a blend of superabsorbent particles comprising a first superabsorbent particles (SAP1) and a second superabsorbent particles (SAP2), wherein the first superabsorbent particles (SAP1) have an AUL that is greater than the AUL of the second superabsorbent particles (SAP2), and wherein the first superabsorbent particles (SAP1) have an AUL of greater than 15 g/g, according to the test method herein, preferably wherein the first superabsorbent particles (SAP1) have a particle size distribution that is greater than that of the second superabsorbent particles (SAP2).

Preferably wherein at least the second superabsorbent particles (SAP2) comprises, preferably consists of, bio-based superabsorbent composite polymer particles comprising a synthetic hydrophobic polymer and a natural biopolymer, more preferably composed of a composite polymer comprising styrene maleic acid copolymer and a biopolymer of animal or vegetal origin; or non-composite polymer particles selected from polyacrylic acid, sodium salt, crosslinked, partly neutralized superabsorbent particles.

Preferably the second superabsorbent particles (SAP2) comprises, preferably consists of, Low-AUL Bio-SAP. "Low-AUL Bio-SAPs" means that they generally have AUL (as measured according to the test method herein) of less than 20 g/g, typically less than 15 g/g, and typically belong to the following classes: (a) materials consisting only of crosslinked biopolymers; (b) materials consisting only of crosslinked synthetic polymers; (c) composite materials consisting of synthetic polymers and biopolymers in certain variations: in inter crosslinking type connection with or without chemical agents; graft type; intercomplexate type and interpenetrate type. Copolymers such as styrene maleic acid are typically used in copolymerization processes for achieving Low-AUL Bio-SAP. Other exemplary sources include alpha-1,3 glucan, starch, starch and/or sodium salts, sugar and derivatives. Exemplary commercially available Low-AUL Bio-SAPs for use herein include: SAPs derived from charged-modified starches as sold from TETHIS 5237 Capital Blvd.; Raleigh, NC 27616, USA and further exemplified in US20200054782 A1; SAPs comprising a 100% acrylic acid co-acrylamide with little to no cross link shell, and the like all generally having AUL of less than 20 g/g according to the test method herein.

Preferably, the first superabsorbent particles (SAP1) are free of Low-AUL Bio-SAP. Advantageously this limits rewet drawbacks as described herein.

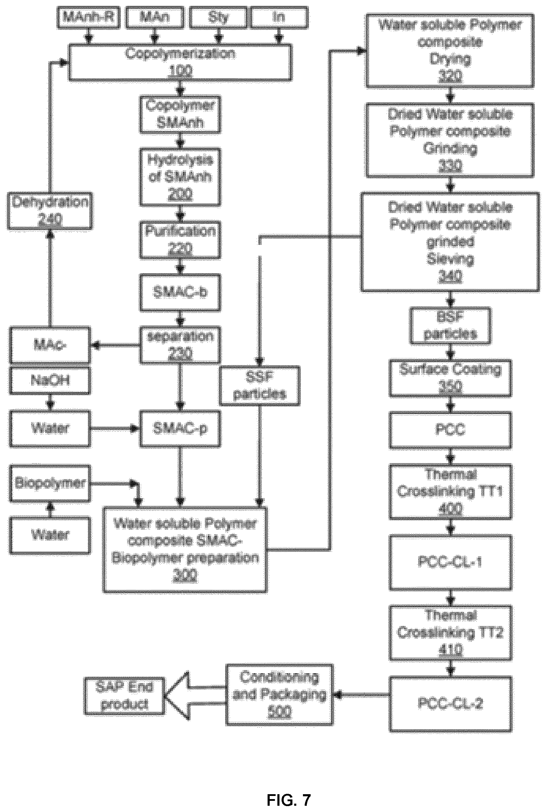

In an embodiment, the SAP1 is a High-AUL Bio-SAP "composite polymer" (i.e. a biodegradable superabsorbent composite polymer having an AUL of greater than 15 g/g, preferably greater than 20 g/g, as will be described in more detail herein below). The composite polymer may comprise a synthetic hydrophobic polymer and a natural biopolymer. More specifically, the composite polymer may comprise styrene maleic acid copolymer and a biopolymer of animal or vegetal origin in conformity with the technological flow chart presented in Fig. 7. Styrene maleic acid copolymer is preferably in salt form, more preferably in the form of monovalent cation salt. Alternatively, the High-AUL Bio-SAP, although less preferred, may comprise "non-composite polymers" and rather be selected from certified biomass superabsorbent polymers having AUL of greater than 15 g/g, preferably greater than 20 g/g, and typically certified by REDcert2 (https://www.redcert.org/images/SP_RC%C2%B2_Biomass-balanced_products_V1.0.pdf ). An example may be a polyacrylic acid, sodium salt, crosslinked, partly neutralized SAP from up to 100% allocated biomass feedstock such as commercially available HySorb® B 6600MB manufactured and sold by BASF SE, Ludwigshafen, Germany.

In an embodiment of the High-AUL Bio-SAP "composite polymer", the styrene moiety in the synthetic polymer can be replaced by other hydrophobic moieties. Exemplary synthetic polymers are: poly(maleic anhydride-co- methyl vinyl ether) (Gantrez), poly(vinyl chloride-co-maleic acid) and poly[(maleic anhydride)- alt-(vinyl acetate).

The term "composite" as herein used refers to a polymeric substance that a) is formed from at least two polymers with different macromolecular chemical structure; and b) the resulting composite is a unique entity that does not separate spontaneously to its components during application. It is understood that the term "composite" may include other substances such as drugs, stimulators, inhibitors, odorants, emollients, plasticizer and others.

The term "anionic" refers to a polymeric composite generating in aqueous media a negative electrochemical potential as the result of the presence in its structure of some free acid functional groups capable of dissociating into anions.

In an embodiment described in Fig. 7, the production process starts with the synthesis of copolymer (styrene-alt-maleic anhydride) by bulk co-polymerization using an excess of about 60-90% of maleic anhydride relative to styrene, whereas maleic anhydride works also as a solvent (operation 100).

Copolymerization is typically executed in Sigma-type mixer machines named Hermetic machines in order to be able to work at high pressures e.g. not exceeding 10 bar or in vacuum conditions (less than IОmbar) with double mantle as well with arms equipped with a heating - cooling system.

The copolymerization process for the production of a superabsorbent polymer typically uses styrene monomer stabilized with organic compounds that inhibit the process of homopolymerization during storage and transportation. Such inhibitors are for example substances such as : amino derivatives, thiols derivatives, and hydroxyl derivates as for example (2-hydroxypropyl)-ethylene diamine compounds, 4-tert-butylcatechol and others) being preferred 4-tert-butylcatechol in proportion of 0.002- 0.008% to the monomer, more preferably is 0.003-0.007% to the styrene and most preferably 0.004-0.006% to the styrene and the molar fraction of styrene in the reaction mass is 0.05-0.08, preferably 0.1-0.15 and more preferably 0.18-0.21.

The copolymerization may also contain maleic anhydride that is either fresh maleic anhydride MAnh or recovered maleic anhydride MAnh-R that is recycled from a previous batch as schematically showed in Fig. 7. and the amount of fresh maleic anhydride MAnh relative to recovered maleic anhydride MAnh-R is about 10 - 40% (dry basis).

For the copolymerization, further customary agents may be used such as peroxides, azo compounds etc., which form free radicals by thermal decomposition, while the quantity of initiator is 0.05- 0.15 %, preferably 0.07-0.009% and most preferably 0.08-0.12% to a double quantity of styrene adopted for copolymerization.

Next, it may follow the conversion of styrene-alt maleic anhydride copolymer to styrene-alt maleic acid copolymer by hydrolysis with water (process 200) which is done in the same equipment where copolymerization has been made. Conversion of styrene-alt maleic anhydride copolymer to styrene-alt maleic acid copolymer by hydrolysis using a quantity of water higher than the one stoichiometrical required for total hydrolysis (Ws) with an excess which represents 2-3% versus to the stoichiometrical value, preferably in excess of 4-5% to the stoichiometrical water and most preferably 5-10% in excess to the stoichiometrical water.

The total quantity of water necessary to styrene and maleic anhydride copolymer's hydrolysis is typically inserted in the mass of reaction in two steps from which 50% is in the form of 0.005N hydrochloric acid solution and the rest as non-acidulated water.

The acidulated water is typically inserted into the reaction mass in two portions at a mass reaction's temperature that not exceeding 60°C at 15 minutes intervals between each dosage, and the quantity of non-acidulated water is inserted into the mass of reaction also in two portions, from which the first is after 60 minutes from the last portion of acidulated water, then is inserted the last portion of non-acidulated water and mixing of reaction mass for 45-60 minutes in cooling conditions of 35-40°C. Reaction mass resulted after hydrolysis is a wet solid in form of a powdery mass of white colour with a value of bulk density of 0.6-0.8 g/cm3. Further, mass of reaction that resulted after copolymerization and hydrolysis (which is a blend of styrene maleic acid copolymer - SMAC and free maleic acid -MAC, which contains traces amounts of non-reacted styrene and stabilizer for styrene as well as traces of hydrochloric acid) is transferred to perform the purification process of styrene maleic acid copolymer (process 220).

The purification process which represents the extraction of free maleic acid fraction from SMAC polymer mass is typically done in an equipment such as tank-type with mixing in which over reaction mass resulted after hydrolysis of synthetic copolymer is added a quantity of deionized water for purification [Wp] that represents a quantity correlated to the wet mass of reactions (RM) in accordance with the relationship Wp= 2 * RM or Wp= 5 * RM, preferably Wp= 3 * RM.

Purification generally consists of 3-5 extraction stages followed each time by filtration. The number of extraction and filtration operations are established so that the content of the free carboxylic groups found in polymer of SMAC to be between 0.00909-0.0095 mole/gram, preferably between 0.0091-0.0094 mole/gram and most preferably between 0.0092- 0.0093 mole/gram.

The extraction in fact preferably occurs at temperature of 60°C for 30 minutes and each filtration (process 230) is done with known equipment as press filter or Nuce filter. All solutions resulted from filtering are collected into a tank of supernatant in order to process the maleic acid which it contain.

The processing of maleic acid water solution to recovery of maleic anhydride preferably consists of the following operations: a) concentration of maleic acid solution through reverse osmosis; b) spray drying of maleic acid concentrated solution when resulted maleic acid powder and water; c) conversion of maleic acid powder to maleic anhydride by thermal-vacuum dehydration (with technological parameters modified than those mentioned in US Pat No.4,414,398 when in the end is obtained a material called recovered maleic anhydride (MAnh-R). The purified filtrate of SMAC polymer is typically collected in an equipment as Sigma mixer type in order to be processed with biopolymers to obtain the water soluble composite polymer containing synthetic SMAC polymer and biopolymer [WSPC] (process 300).

The preparation process of polymeric composite [WSPC] containing SMAC and a biopolymer of animal or vegetal origin is preferably preceded by other operations as follows: a) Preparation of a base solution is obtained by dissolution of a solid hydroxide compound in water to 40% by weight, whereas examples of preferred base hydroxide compounds are sodium hydroxide, lithium hydroxide, potassium hydroxide, ammonium hydroxide, preferably sodium hydroxide; b) transformation of styrene maleic acid copolymer obtained in step 230 into styrene-maleic acid monovalent cation salt by neutralization with base solution prepared in a) above in order to obtain a solution of copolymer salt with concentration higher than 25% preferably higher than 35 % and most preferably higher than 50%. Neutralization of the SMAC copolymer is 48-58%, preferably 50-56% and most preferably 52-54%; c) preparation of the biopolymer (as gelatin, albumin, casein, soy, guar or starch, preferably is gelatin) as water solution of 40% by weight in water; d) preparation of polymeric composite is done by treating the solution of styrene maleic acid salt with biopolymer solution at temperature of 55-75°C for 30 minutes.

The amount of biopolymer relative to copolymer in the composite is preferably from 4-6 % (dry basis), preferably 8-10 % (dry basis) and most preferably 12-14 % (dry basis).

The blending of the composite mass typically continues during 4-5 hours until the polymeric mass is transformed from the viscous solution into a partially dried granular mass with a moisture content not higher than 20%. This partially dried polymeric composite material WSPC in granular form is typically subjected to a supplementary drying (process 320) at temperatures of preferably 75-85°C on conveyor belt type or Rotary type in order to obtain final drying of until the moisture content is less than 14%, preferably less than 12% and most preferably less than 8%.

Further steps of drying, grinding and sieving are preferably carried out (process 330 + process 340) to obtain two types of solid phases called herein large solid phase (LSP) with granulometric distribution higher than 100 microns, preferably of 100 -850 microns that corresponds to be used in the manufacture of diapers and a small solid phase (SSF) with granulometric distribution up to 100 microns. The small solid phase SSF is re-used in the preparation of the next new batch of SAP that comes into the process 300.

The large solid phase LSP may be exposed to post-treatment surface coating processes using known chemical substances as: glycerin ethylene glycol, propylene glycol or polyether hydroxyl with properties of biodegradability. Examples of preferred coating materials are hydroxyl polyether, more preferably polyethylene glycol - PEG 200 used at a rate of 0.2-2% by weight (dry basis) to LSP, preferably at a rate of 0.5-1.5% by weight and most preferably at a rate of 0.8-1.2% by weight to LSP.

Surface coating may be applied using equipment like powder coating machines at temperatures of generally 30-70°C, preferably at temperatures of 35-65°C and most preferably at temperatures of 40-60°C for 30 -90 minutes, preferably for 40-75 minutes and most preferably for 50-60 minutes. As a result of this process is resulted the material called Polymer Composite Coated Treated- PCC (operation 350).

The obtained material after surface coating (Polymer Composite Coated) may be subdue to a thermal treatment called first thermal treatment (TT1) (operation 400) consisting in warming of particles mass in hot air with temperature of 90-l40°C for 30-150 minutes, preferably with temperatures of 100-135°C for 45-120 minutes and most preferably by bulk thermal crosslinking at temperatures of 110-120°C, for 60-90 minute using equipment of conveyor belt type or shaking rotary type when is resulted an intermediary material called Polymer Composite Coated first crosslinked (PCC-CL-I).

The material PCC-CL-I may be subdue to a new thermal treatment (TT2) (operation 410) in hot air with temperature of 120-150°C for 5-30 minutes, preferably at temperatures of 125-145°C for 10-25 minutes and most preferably to temperature of 120-140°C for 15-20 minute in the same type of equipment used for TT1 and is obtained the Polymer Composite Coated second crosslinked (PCC-CL-2). The material (PCC-CL-2) may then be conditioned for 24 hours in an atmosphere in which the air has a moisture content of 65% and temperature of 20°C and then is packaged in sealed polyethylene bags (operation 500).

The material resulting after conditioning is a biodegradable SAP with AUL higher than 20g/g in aqueous solution of 0.9% NaCl at pressure of 0.9 psi, which represents the end product of the manufacturing process which is the object of the present invention, i.e. the "High-AUL biodegradable superabsorbent composite polymer" referred to herein.

Reference is made to co-pending application EP21152698.3 for further examples of Low-AUL Bio-SAP and High-AUL Bio-SAPs that may be used in absorbent cores herein, with particular reference to Example 1.

In an embodiment, the first superabsorbent particles (SAP1) are comprised at a level of less than 80%wt, preferably from 10%wt to 32%wt; and the second superabsorbent particles (SAP2) are comprised at a level of at least 20%wt, preferably from 25%wt to 100%wt, more preferably from 30% to 90%, even more preferably from 35% to 80%, even more preferably from 40% to 70%, even more preferably from 45% to 68%, by total weight of superabsorbent particles.

Preferably, the AUL ratio AULSAP1/AULSAP2 of the first superabsorbent particles (SAP1) and second superabsorbent particles (SAP2) is greater than 1.4, preferably greater than 1.5, more preferably from 1.6 to 5, even more preferably from 1.7 to 3. Advantageously, this allows to ensure the right balance of reduced rewet and fast liquid absorption especially on the lower layer and optimal performance under load.

In an embodiment, the first superabsorbent particles (SAP1) have a lower absorption speed (typically according to the vortex method herein) than the second superabsorbent particles (SAP2). Preferably, the vortex time (according to the vortex method herein) of SAP1 is more than 50 seconds, preferably from 60 seconds to 90 seconds. Preferably, the absorption speed of SAP2 is less than 45 seconds, preferably less than 40 seconds, even more preferably from 15 seconds to 35 seconds.

The absorbent inserts herein preferably comprise a wetness indicator which is visible from the exterior of the insert and/or article and which changes appearance when contacted with a body exudates, in particular urine. The wetness indicator may be placed, when seen from the exterior of the insert and/or article, between the two channel-forming areas and/or channels and/or attachment zones according to some embodiments herein.

The wetness indicator serves the role of alerting the wearer that the insert has been soiled and may need changing.

The wetness indicators for use herein may be according to any wetness indicating system known in the art. It is known that wetness indicator can provide an appearing signal, a disappearing signal or a colour change signal, and of course combinations thereof. An appearing signal will typically not be visible or more generally perceivable in the dry insert, and becomes visible or otherwise perceivable when the insert is wet. An appearing signal may for example be provided by a composition which is transparent or having a colour that matches the colour of the backsheet material, which is typically white, in its dry state, and then changes to a different colour when contacted with urine. Other appearing wetness indicator may also be elements capable of providing a physical sensation indicating a fullness level of the absorbent assembly. Examples of such elements are disclosed in WO2008132630 and include a temperature change element (cooling or heating element), a pressure-inducing element or a foam-producing element.

The wetness indicator composition may be applied to a layer of the absorbent insert as described herein using a conventional technique, for example printing, spraying or coating, during the making of the absorbent insert. The layer may advantageously be the inner surface of the backsheet or the outer surface of the bottom side of the core wrap or both. This allows the wetness indicator to be visible from the exterior of the insert by transparency through the backsheet while keeping the wetness indicator composition within the insert. The wetness indicator may in particular be easily applied on a layer such a nonwoven or film by a slot-coating process especially if the composition is can be applied as a hotmelt. The slot-coating process allows applying a well-defined slot or a series of slots extending in the machine direction of the converting line, which is typically parallel to the longitudinal direction of the insert.

In an embodiment, the absorbent core (4) comprises a core wrap enclosing the absorbent material (5) therein, and wherein the wetness indicator is positioned between a garment-facing side of the core wrap and a body-facing surface of the backsheet (3), preferably positioned between a garment-facing side of the bottom/lower core wrap (15) and a body-facing surface of the backsheet (3).

Preferably, the wetness indicator extends along the longitudinal centerline (y) and may cross the transverse centerline (x), wherein the wetness indicator extends from about the transverse centerline (x) (i.e. from a position proximal to the transverse centerline (x) or a position being on the centeriline (x)) to a first terminal position in a direction towards the first transverse edge (6) and extends from about the transverse centerline (x) to a second terminal position in a direction towards the second transverse edge (7); wherein the distance between the first terminal position and the first transverse edge (6) is less than the distance between the second terminal position and the second transverse edge (7) or vice versa. Advantageously this allows for accurate location/fitting of the insert when a specific front-to-back relationship is required. Indeed the fact that the wetness indicator is visually asymmetrically oriented about the transverse centerline allows for creating indicia for the user on the correct front/back positioning to place onto the corresponding outer shell, especially when the outer shell comprises a corresponding window and/or sensor having substantially the same shape of the wetness indicator which need to be overlayed one on top of the other.

Preferably, the wetness indicator has a first maximum width and the window (23) has a second maximum width (W), generally extending along a direction substantially parallel to the transverse centerline (x), and wherein the first maximum width is less than the second maximum width (W). Advantageously this allows for the full wetness indicator to be viewable from the garment facing side of the outer shell (20) when connected thereto.

Preferably the wetness indicator has a first maximum width being greater than 3mm, preferably from 5mm to 30mm, even more preferably from 10mm to 20mm. Advantageously a wider than normal wetness indicator is advantageous for increased ease of viewing through the outer shell (and/or detection by the optical sensor as described herein), however higher widths do not provide significant improvement in visibility and rather add material and cost.

In an embodiment, the absorbent core (4) comprises one or more attachment zones wherein a top layer of a core wrap (14) is adhered to a lower layer of a core wrap (15) such that one or more channels (13) substantially free of absorbent material are formed, preferably wherein the wetness indicator extends between said channels (13) and preferably overlaps at least a portion of said channels. The channels (13) may extend from 10% to 85%, preferably from 15% to 75%, of a length of the core (4) extending in a direction substantially parallel to the longitudinal axis (y).

Preferably, the absorbent core (4) comprises one or more attachment zones wherein the top layer (14) of the core wrap is adhered to the lower layer (15) of the core wrap such that one or more channels (13) substantially free of absorbent material are formed, preferably inboard of a perimeter of said core (4) so that said channels (13) do not extend to or reach said perimeter. Advantageously this allows to reduce the list of leakage as well as cooperate with the folds to provide improved cup formation when worn.

In a preferred embodiment, channels (13) herein are longitudinally extending and cross both the transverse centerline (x) at a first crossing point and the longitudinal centerline (y) at a second crossing point, wherein the first and second crossing points are different and preferably wherein the second crossing point is positioned closer to the second transverse edge (7) than the first crossing point, and preferably wherein the second transverse edge (7) is positioned proximal to the back region (B) of the outer shell (20) when said insert (1) is connected thereto. Advantageously this allows for improved cup formation in a hybrid concept as well as retaining liquid distribution advantages by guiding exudates to the back of the core.

THE ABSORBENT ASSEMBLY

Absorbent assemblies herein may comprise an insert (1) as described herein coupled to an outer shell (20) as described herein.

There may be provided a kit of parts comprising: a plurality of inserts (1) as described herein; and one or more outer shells (20) as described herein. Such kits may allow for extended use of the re-usable outer shells.

Preferably, the plurality of inserts (1) differ in at least one of: size, absorbency, biodegradable and/or compostable content, shape, and number of components contained therein. Advantageously this allows for different inserts targeting different types of usage e.g. day vs night, size increases of subjects/wearers etc. whilst retaining substantially the same outer shell.

METHOD OF MANUFACTURE

Methods for the manufacture of disposable absorbent inserts herein comprise the steps of:

(i) providing a first web of material, preferably a liquid permeable nonwoven;
(ii) depositing absorbent material onto said first web;
(iii) providing a second web of material, preferably a liquid permeable nonwoven, and applying said second web of material over the deposited absorbent material, or folding said first web to enclose the deposited absorbent material therein;
(iv) joining the first and second webs together, or the folded first web, at the one or more attachment areas to form an absorbent core comprising absorbent material enclosed by said web(s);
(v) optionally applying an acquisition distribution layer;
(vi) sandwiching the absorbent core, and preferably the acquisition distribution layer, between a liquid permeable layer and a substantially liquid impermeable layer oppositely disposed thereto, preferably with the acquisition distribution layer being in contact with the liquid permeable layer, to form a disposable absorbent insert;

wherein after step (vi) a first folding step is applied wherein first and second longitudinal edges of said insert are tucked-in by folding said edges about a folding axis running parallel to a machine direction such that longitudi-

nally extending folds ($F_o$, $F_o'$) proximal to each said first and second longitudinal edges (8, 9) are formed wherein said folds ($F_o$, $F_o'$) comprise a contact surface where a first portion of the substantially liquid impermeable layer forming a backsheet (3) is in contact with a second portion of said backsheet (3) and wherein said contact surface is a garment-facing surface of said backsheet (3). Advantageously this method allows for the formation of inserts that reduce the footprint thereof as well as importantly create a stiffening structure at the longitudinal edges for ease of handling and coupling to a re-usable outer shell, this without the need of added stiffening structures to be incorporated.

In an embodiment the folding step is carried out statically (i.e. without the use of rotating or moving elements) and typically comprising one or more stators arranged to fold the respective sections of the insert laminate as it is guided therealong and/or therethrough.

Preferably, after the fist folding step at least a first pressure is applied, for example by one or more pressure rollers. The pressure may be selectively applied substantially only over the folds outboard of a central section comprising the absorbent material, for example by at least two oppositely positioned pressure rollers at a distance from each other along an axis perpendicular to the (z)-axis and parallel to the longitudinal axis (y). Advantageously this allows added consolidating of the folds whilst limiting damage to the absorbent core (e.g. inadvertent crushing of absorbent polymer particles when present or piercing the topsheet and/or backsheet).

In an embodiment, prior to the first folding step an adhesive is applied in one or more continuous or discontinuous stripes along at least a portion of the longitudinally extending flanges ($F_L$, $F_L'$), or an area adjacently inboard thereof, on a garment-facing surface thereof such that, upon folding, the flanges ($F_L$, $F_L'$) are joined to the garment-facing surface of the backsheet. In addition or alternatively, during or after the first folding step the folded flanges ($F_L$, $F_L'$) are mechanically bonded such that said flanges ($F_L$, $F_L'$) are joined to a garment-facing surface of the backsheet at a plurality of discrete bonding joints. Typically such mechanical bonding is selected by heat and/or pressure bonding or ultrasonic bonding. Advantageously this allows for further resistance to unfolding which may bring about further benefits especially in terms of mechanical resistance to bending in yet a cost-effective manner.

In an embodiment, after the first folding step a second folding step is applied wherein the insert is folded about a transverse centerline (x) such that the longitudinally extending folds ($F_o$, $F_o'$) substantially enclose other portions of the folded insert therein. In such manner the inserts may then be compacted and effectively stacked for packaging.

Preferably, the method comprises a cutting step after the first folding step to cut the laminated structure into a plurality of disposable absorbent inserts. Preferably wherein the second folding step occurs sequentially after the cutting step.

TEST METHODS

AUL (Absorbency Under Load, 0.7 psi)

Absorbency Under Load is determined similarly to the absorption under pressure test method No. 442.2-02 recommended by EDANA (European Disposables and Nonwovens Association), except that for each example the actual sample having the particle size distribution reported in the example is measured.

The measuring cell for determining AUL 0.7 psi is a Plexiglas cylinder 60 mm in internal diameter and 50 mm in height. Adhesively attached to its underside is a stainless steel sieve bottom having a mesh size of 36 $\mu$m. The measuring cell further includes a plastic plate having a diameter of 59 mm and a weight which can be placed in the measuring cell together with the plastic plate. The weight of the plastic plate and the weight together weigh 1345 g. AUL 0.7 psi is determined by determining the weight of the empty Plexiglas cylinder and of the plastic plate and recording it as WO. Then 0.900 +/- 0.005 g of water-absorbing polymer or material (particle size distribution 150 - 800 $\mu$m or as specifically reported in the examples which follow) is weighed into the Plexiglas cylinder and distributed very uniformly over the stainless steel sieve bottom. The plastic plate is then carefully placed in the Plexiglas cylinder, the entire unit is weighed and the weight is recorded as Wa. The weight is then placed on the plastic plate in the Plexiglas cylinder. A ceramic filter plate 120 mm in diameter, 10 mm in height and 0 in porosity (Duran, from Schott) is then placed in the middle of the Petri dish 200 mm in diameter and 30 mm in height and sufficient 0.9% by weight sodium chloride solution is introduced for the surface of the liquid to be level with the filter plate surface without the surface of the filter plate being wetted. A round filter paper 90 mm in diameter and < 20 $\mu$m in pore size (S&S 589 Schwarzband from Schleicher & Schüll) is subsequently placed on the ceramic plate. The Plexiglas cylinder holding the material or polymer is then placed with the plastic plate and weight on top of the filter paper and left there for 60 minutes. At the end of this period, the complete unit is taken out of the Petri dish from the filter paper and then the weight is removed from the Plexiglas cylinder. The Plexiglas cylinder holding swollen water-absorbing material or polymer is weighed out together with the plastic plate and the weight is recorded as Wb.

Absorbency under load (AUL) is calculated as follows:

$$AUL0.7psig/g=Wb-Wa/Wa-W0$$

AUL 0.3 psi and 0.5 psi are measured similarly at the appropriate lower pressure.

Absorption speed (Vortex) measurement:

The vortex test measures the amount of time in seconds required for 2 grams of a superabsorbent material to close a vortex created by stirring 50 milliliters of saline solution at 600 revolutions per minute on a magnetic stir plate. The time it takes for the vortex to close (that the surface of the fluid becomes flat meaning that in the beginning, the centrifugal force that is caused by the rotation of the fluid creates a "coning-in" in the surface, but when the gelling of the SAP proceeds the viscosity of the fluid increases so that the depth of the indentation decreases until it's finally substantially flat) is an indication of the free swell absorbing rate of the superabsorbent material.

Equipment and materials:

1. Beaker, 100 ml.
2. Programmable magnetic stir plate, capable of providing 600 revolutions per minute.
3. Magnetic stir bar without rings, 7.9 mm $\times$ 32 mm, Teflon covered.
4. Stopwatch.
5. Balance, accurate to $\pm$ 0.01 g.
6. Saline solution, 0.9%.
7. Weighing paper.
8. Room with standard condition atmosphere: Temp = 23°C $\pm$ 1°C and Relative Humidity = 50% $\pm$ 2%.

Test procedure:

1. Measure 50 g $\pm$ 0.01 g of saline solution into the 100 ml beaker.
2. Place the magnetic stir bar into the beaker.
3. Program the magnetic stir plate to 600 revolutions per minute.
4. Place the beaker on the center of the magnetic stir plate such that the magnetic stir bar is activated. The bottom of the vortex should be near the top of the stir bar.
5. Weigh out 2 g $\pm$ 0.01 g of the superabsorbent material to be tested on weighing paper.
6. While the saline solution is being stirred, pour the superabsorbent material to be tested into the saline solution and start the stopwatch. The superabsorbent material to be tested should be added to the saline solution between the center of the vortex and the side of the beaker.
7. Stop the stopwatch when the surface of the saline solution becomes flat, and record the time.
8. The time, recorded in seconds, is reported as the Vortex Time.

It is supposed that the present invention is not restricted to any form of realization described previously and that some modifications can be added to the presented embodiments without reappraisal of the appended claims.

**Claims**

1. A disposable absorbent insert (1) comprising:

    a liquid permeable topsheet (2);
    a liquid impermeable backsheet (3); and
    an absorbent core (4) comprising absorbent material (5) therein and being sandwiched between said topsheet (2) and backsheet (3);
    wherein the insert (1) comprises a perimeter formed by first and second transverse edges (6, 7) and first and second longitudinal edges (8, 9) connecting the first and second transverse edges (6, 7); a longitudinal centerline (y) extending substantially parallel to said first and second longitudinal edges (8, 9) and interposed therebetween such to divide said insert (1) into a first longitudinal half between said longitudinal centerline (y) and said first longitudinal edge (8) and a second longitudinal half between said longitudinal centerline (y) and said second longitudinal edge (9); and a transverse centerline (x) extending substantially parallel to said first and second transverse edges (6, 7) and interposed therebetween such to divide said insert (1) into a first transverse half between said transverse centerline (x) and said first transverse edge (6) and a second transverse half between said transverse centerline (x) and said second transverse edge (7);
    **characterised in that** the insert (1) comprises longitudinally extending folds ($F_o$, $F_o'$) proximal to each said first and second longitudinal edges (8, 9) wherein said folds ($F_o$, $F_o'$) comprise a contact surface where a first portion of the backsheet (3) is in contact with a second portion of the backsheet (3) and **in that** said contact surface is a garment-facing surface of said backsheet (3).

2. An insert (1) according to Claim 1 wherein said insert comprises oppositely disposed longitudinally extending flanges ($F_L$, $F_L'$) along the first and second longitudinal edges (8, 9) and wherein said flanges ($F_L$, $F_L'$) are folded to form the longitudinally extending folds ($F_o$, $F_o'$), preferably wherein the longitudinally extending flanges ($F_L$, $F_L'$) are substantially free of absorbent material.

3. An insert (1) according to Claim 2 wherein the longitudinally extending flanges ($F_L$, $F_L'$) comprise the topsheet (2) and the backsheet (3) being directly or indirectly joined to each other.

4. An insert (1) according to any of the preceding Claims comprising at least a pair of lateral cuffs ($C_F$, $C_F'$) wherein at least one of said cuffs is positioned along at least a portion of the first longitudinal edge (8) and at least one of said cuffs is positioned along at least a portion of the second longitudinal edge (9), preferably wherein the cuffs comprise one or more elastics (e, e') proximal to an apex thereof such that a tension thereof causes the cuffs to project upwards away from the topsheet (2).

5. An insert (1) according to Claim 4 wherein the cuffs are positioned substantially inboard of longitudinally extending flanges ($F_L$, $F_L'$).

6. An insert (1) according to any of the preceding Claims wherein the absorbent core (4) comprises a core wrap comprising a top layer (14) and a bottom layer (15) and wherein the absorbent material (5) is enclosed by said core wrap between said top and bottom layers (14, 15) of the core wrap.

7. An insert (1) according to Claim 6 wherein the absorbent core (4) comprises one or more attachment zones wherein the top layer (14) of the core wrap is adhered to the lower layer (15) of the core wrap such that one or more channels (13) substantially free of absorbent material are formed, preferably inboard of a perimeter of said core (4) so that said channels (13) do not extend to or reach said perimeter.

8. An absorbent assembly comprising:

   an insert (1) according to any of Claims 1 to 7; and
   a re-usable outer shell (20).

9. An absorbent assembly according to Claim 8 wherein the re-usable outer shell (20) comprises:

   a front (F) and back (B) waist region having uppermost and lowermost edges; and
   a crotch region (C) interposed between the front (F) and back (B) waist region,
   wherein the uppermost edges of the waist region form a waist opening and the lowermost edges of the waist region together with lateral extremities of the crotch region form two oppositely disposed leg openings;
   and wherein at least the body-facing surface of the crotch region (C) is adapted for receiving an absorbent insert (1) according to any of the preceding claims.

10. An absorbent assembly according to Claim 9 comprising re-fastenable or permanent side seams joining the front and back waist regions, said outer shell (20) preferably being in the form of a pant.

11. A kit of parts comprising:

    a plurality of inserts (1) according to any of Claims 1 to 7; and
    one or more outer shells (20) according to any of Claims 8 to 10.

12. A kit according to Claim 11 wherein the plurality of inserts (1) differ in at least one of: size, absorbency, biodegradable and/or compostable content, shape, and number of components contained therein.

13. An insert (1) according to any of the preceding Claims further comprising an acquisition distribution layer (ADL) positioned between the topsheet (2) and the absorbent core (4).

14. An insert (1) according to any of the preceding Claims wherein the topsheet (2) is a nonwoven comprising plant-based fibers wherein said plant-based fibers comprise, preferably consist of, harvested fibers other than wood pulp, such that said topsheet (2) has a bio-based content of from about 10% to about 100% using ASTM D6866-10, method B, and optionally wherein said nonwoven further comprises synthetic fibers generally in an amount to provide added structural integrity such as from 10% to 60% by weight of said nonwoven.

15. An insert (1) according to any of the preceding Claims wherein the backsheet (3) comprises bio-PE, and where the absorbent material comprises superabsorbent polymer particles preferably selected from Low-AUL Bio-SAP having an AUL less than 15 g/g and High-AUL Bio-SAP having an AUL greater than 15 g/g, as measured according to the test method of the description

16. An insert (1) according to any of the preceding Claims wherein at least 85%, preferably at least 90%wt, more preferably at least 95%wt, by total weight of the disposable absorbent insert (1) comprises components having a bio-based content of from about 10% to about 100%, preferably from about 15% to about 100%, even more preferably from about 20% to about 100%, according to ASTM D6866-10, method B.

17. A method for the manufacture of disposable absorbent inserts, preferably according to any of the preceding Claims, said method comprising the steps of:

    (i) providing a first web of material, preferably a liquid permeable nonwoven;

(ii) depositing absorbent material onto said first web;

(iii) providing a second web of material, preferably a liquid permeable nonwoven, and applying said second web of material over the deposited absorbent material, or folding said first web to enclose the deposited absorbent material therein;

(iv) joining the first and second webs together, or the folded first web, at the one or more attachment areas to form an absorbent core comprising absorbent material enclosed by said web(s);

(v) optionally applying an acquisition distribution layer;

(vi) sandwiching the absorbent core, and preferably the acquisition distribution layer, between a liquid permeable layer and a substantially liquid impermeable layer oppositely disposed thereto, preferably with the acquisition distribution layer being in contact with the liquid permeable layer, to form a disposable absorbent insert;

**characterized in that** after step (vi) a first folding step is applied wherein first and second longitudinal edges of said insert are tucked-in by folding said edges about a folding axis running parallel to a machine direction such that longitudinally extending folds ($F_o$, $F_o'$) proximal to each said first and second longitudinal edges (8, 9) are formed wherein said folds ($F_o$, $F_o'$) comprise a contact surface where a first portion of the substantially liquid impermeable layer forming a backsheet (3) is in contact with a second portion of said backsheet (3) and **in that** said contact surface is a garment-facing surface of said backsheet (3).

18. A method according to Claim 16 wherein after the first folding step a second folding step is applied wherein the insert is folded about a transverse centerline (x) such that the longitudinally extending folds ($F_o$, $F_o'$) substantially enclose other portions of the folded insert therein.

**Patentansprüche**

1. Saugfähige Einwegeinlage (1), umfassend:

eine flüssigkeitsdurchlässige Decklage (2);
eine flüssigkeitsundurchlässige Rückseitenlage (3);
einen saugfähigen Kern (4), der saugfähiges Material (5) umfasst und sandwichartig zwischen der Decklage (2) und der Rückseitenlage (3) eingelegt ist;
wobei die Einlage (1) einen Umfang umfasst, der durch erste und zweite Querränder (6, 7) und erste und zweite Längsränder (8, 9) gebildet wird, welche die ersten und zweiten Querränder (6, 7) verbinden; eine Längsmittellinie (y), die sich im Wesentlichen parallel zu den ersten und zweiten Längsrändern (8, 9) erstreckt, und derart dazwischen angeordnet ist, um die Einlage (1) in eine erste Längshälfte zwischen der Längsmittellinie (y) und dem ersten Längsrand (8) und eine zweite Längshälfte zwischen der Längsmittellinie (y) und dem zweiten Längsrand (9) zu teilen; und eine Quermittellinie (x), die sich im Wesentlichen parallel zu den ersten und zweiten Querrändern (6, 7) erstreckt und derart dazwischen angeordnet ist, um die Einlage (1) in eine erste Querhälfte zwischen der Quermittellinie (x) und dem ersten Querrand (6) und eine zweite Querhälfte zwischen der Quermittellinie (x) und dem zweiten Querrand (7) zu teilen; **dadurch gekennzeichnet, dass** die Einlage (1) sich längs erstreckende Falten ($F_o$, $F_o'$) proximal zu jedem ersten und zweiten Längsrand (8, 9) umfasst, wobei die Falten ($F_o$, $F_o'$) eine Kontaktoberfläche umfassen, an der ein erster Abschnitt der Rückseitenlage (3) mit einem zweiten Abschnitt der Rückseitenlage (3) in Kontakt ist, und **dadurch, dass** die Kontaktoberfläche eine der Kleidung zugewandte Oberfläche der Rückseitenlage (3) ist.

2. Einlage (1) nach Anspruch 1, wobei die Einlage gegenüber angeordnete, sich längs erstreckende Flansche ($F_L$, $F_L'$) entlang des ersten und zweiten Längsrandes (8, 9) umfasst und wobei die Flansche ($F_L$, $F_L'$) gefaltet sind, um die sich längs erstreckenden Falten ($F_o$, $F_o'$) zu bilden, wobei die sich längs erstreckenden Flansche ($F_L$, $F_L'$) vorzugsweise im Wesentlichen frei von saugfähigem Material sind.

3. Einlage (1) nach Anspruch 2, wobei die sich längs erstreckenden Flansche ($F_L$, $F_L'$) die Decklage (2) und die Rückseitenlage (3) umfassen, die direkt oder indirekt miteinander verbunden sind.

4. Einlage (1) nach einem der vorstehenden Ansprüche, die mindestens ein Paar seitliche Bündchen ($C_F$, $C_F'$) umfasst, wobei mindestens eines dieser Bündchen entlang mindestens eines Abschnitts des ersten Längsrandes (8) angeordnet ist und mindestens eines dieser Bündchen entlang mindestens eines Abschnitts des zweiten Längsrandes (9) angeordnet ist, wobei die Bündchen vorzugsweise ein oder mehrere Gummibänder (e, e') proximal zu einem Scheitelpunkt davon umfassen, sodass eine Spannung davon die Bündchen veranlasst, nach oben weg von der Decklage (2) zu ragen.

5. Einlage (1) nach Anspruch 4, wobei die Bündchen im Wesentlichen innerhalb der sich längs erstreckenden Flansche ($F_L$, $F_L'$) positioniert sind.

6. Einlage (1) nach einem der vorstehenden Ansprüche, wobei der saugfähige Kern (4) eine Kernumhüllung umfasst, die eine obere Lage (14) und eine untere Lage (15) umfasst, und wobei das saugfähige Material (5) von der Kernumhüllung zwischen der oberen und unteren Lage (14, 15) der Kernumhüllung umschlossen ist.

7. Einlage (1) nach Anspruch 6, wobei der saugfähige Kern (4) eine oder mehrere Befestigungszonen umfasst, wobei die obere Lage (14) der Kernumhüllung an der unteren Lage (15) der Kernumhüllung anhaftet, sodass ein oder mehrere Kanäle (13) im Wesentlichen frei von saugfähigem Material vorzugsweise innerhalb eines Umfangs des Kerns (4) gebildet werden, sodass sich die Kanäle (13) nicht bis zum Umfang erstrecken oder diesen erreichen.

8. Saugfähige Anordnung, umfassend:

   eine Einlage (1) nach einem der Ansprüche 1 bis 7; und
   eine wiederverwendbare Außenhülle (20).

9. Saugfähige Anordnung nach Anspruch 8, wobei die wiederverwendbare Außenhülle (20) umfasst:

   einen vorderen (F) und hinteren (B) Taillenbereich, der oberste und unterste Ränder aufweist; und einen Schrittbereich (C), der zwischen dem vorderen (F) und hinteren (B) Taillenbereich angeordnet ist, wobei die obersten Ränder des Taillenbereichs eine Taillenöffnung bilden und die untersten Ränder des Taillenbereichs zusammen mit seitlichen Enden des Schrittbereichs zwei gegenüber angeordnete Beinöffnungen bilden;
   und wobei zumindest die dem Körper zugewandte Oberfläche des Schrittbereichs (C) zur Aufnahme einer saugfähigen Einlage (1) nach einem der vorstehenden Ansprüche angepasst ist.

10. Saugfähige Anordnung nach Anspruch 9, die wiederbefestigbare oder permanente Seitennähte aufweist, welche die vorderen und hinteren Taillenbereiche verbinden, wobei die Außenhülle (20) vorzugsweise in Form einer Hose ist.

11. Kit aus Teilen, umfassend:

   eine Vielzahl von Einlagen (1) nach einem der Ansprüche 1 bis 7; und
   eine oder mehrere Außenhüllen (20) nach einem der Ansprüche 8 bis 10.

12. Kit nach Anspruch 11, wobei sich die Vielzahl der Einlagen (1) in mindestens einem unterscheiden von: Größe, Saugfähigkeit, biologisch abbaubarem und/oder kompostierbarem Inhalt, Form und Anzahl von darin enthaltenen Komponenten.

13. Einlage (1) nach einem der vorstehenden Ansprüche, die weiter eine zwischen der Decklage (2) und dem saugfähigen Kern (4) positionierte Aufnahme-Verteilungslage (ADL) umfasst.

14. Einlage (1) nach einem der vorstehenden Ansprüche, wobei die Decklage (2) ein Vliesstoff ist, der Fasern auf Pflanzenbasis umfasst, wobei die Fasern auf Pflanzenbasis andere geerntete Fasern als Zellstoff umfassen, vorzugsweise daraus bestehen, sodass die Decklage (2) einen biobasierten Gehalt von etwa 10% bis etwa 100% gemäß ASTM D6866-10, Verfahren B, aufweist, und wobei der Vliesstoff optional weiter synthetische Fasern im Allgemeinen in einer Menge umfasst, um zusätzliche strukturelle Integrität bereitzustellen, wie beispielsweise 10 Gew.-% bis 60 Gew.-% des Vliesstoffs.

15. Einlage (1) nach einem der vorstehenden Ansprüche, wobei die Rückseitenlage (3) Bio-PE umfasst und wobei das saugfähige Material supersaugfähige Polymerpartikel umfasst, die vorzugsweise aus Low-AUL-Bio-SAP, das einen AUL-Wert von weniger als 15 g/g aufweist, und High-AUL-Bio-SAP, das einen AUL-Wert von mehr als 15 g/g aufweist, wie nach dem Testverfahren der Beschreibung gemessen, ausgewählt sind.

16. Einlage (1) nach einem der vorstehenden Ansprüche, wobei mindestens 85 Gew.-%, vorzugsweise mindestens 90 Gew.-%, bevorzugter mindestens 95 Gew.-%, bezogen auf das Gesamtgewicht der saugfähigen Einwegeinlage (1) Komponenten umfassen, die einen biobasierten Gehalt von etwa 10% bis etwa 100%, vorzugsweise von etwa 15% bis etwa 100%, noch bevorzugter von etwa 20% bis etwa 100%, gemäß ASTM D6866-10, Verfahren B, aufweisen.

17. Verfahren zur Herstellung von saugfähigen Einwegeinlagen, vorzugsweise nach einem der vorstehenden Ansprüche, wobei das Verfahren die Schritte umfasst zum:

   (i) Bereitstellen einer ersten Materialbahn, voreugsweise eines flüssigkeitsdurchlässigen Vlieses;
   (ii) Aufbringen eines saugfähigen Materials auf die erste Bahn;
   (iii) Bereitstellen einer zweiten Materialbahn, vorzugsweise eines flüssigkeitsdurchlässigen Vlieses, und Anlegen der zweiten Materialbahn über dem aufgebrachten saugfähigen Material oder Falten der ersten Bahn, um das aufgebrachte saugfähige Material darin einzuschließen;

(iv) Verbinden der ersten und zweiten Bahn oder der gefalteten ersten Bahn an dem einen oder mehreren Befestigungsbereichen miteinander, um einen saugfähigen Kern zu bilden, der saugfähiges Material umfasst, das von der/den Bahn(en) umschlossen ist;

(v) optional Anlegen einer Aufnahme-Verteilungslage;

(vi) sandwichartig Einlegen des saugfähigen Kerns und vorzugsweise der Aufnahme-Verteilungsschicht zwischen einer flüssigkeitsdurchlässigen Lage und einer gegenüber angeordneten, im Wesentlichen flüssigkeitsundurchlässigen Lage, wobei die Aufnahme-Verteilungsschicht vorzugsweise in Kontakt mit der flüssigkeitsdurchlässigen Lage ist, um eine saugfähige Einwegeinlage zu bilden;

**dadurch gekennzeichnet, dass** nach Schritt (vi) ein erster Faltschritt angewendet wird, wobei erste und zweite Längsränder der Einlage durch Falten der Ränder um eine Faltachse, die parallel zu einer Maschinenrichtung verläuft, eingeschlagen werden, sodass sich längs erstreckende Falten ($F_o$, $F_o'$) proximal zu jedem des ersten und zweiten Längsrandes (8, 9) bilden, wobei die Falten ($F_o$, $F_o'$) eine Kontaktoberfläche umfassen, wo ein erster Abschnitt der im Wesentlichen flüssigkeitsundurchlässigen Lage, die eine Rückseitenlage (3) bildet, mit einem zweiten Abschnitt der Rückseitenlage (3) in Kontakt ist, und **dadurch, dass** die Kontaktoberfläche eine der Kleidung zugewandte Oberfläche der Rückseitenlage (3) ist.

18. Verfahren nach Anspruch 16, wobei nach dem ersten Faltschritt ein zweiter Faltschritt angewendet wird, wobei die Einlage um eine Quermittellinie (x) gefaltet wird, sodass die sich längs erstreckenden Falten ($F_o$, $F_o'$) andere Abschnitte der gefalteten Einlage im Wesentlichen darin einschließen.

**Revendications**

1. Insert absorbant jetable (1) comprenant :

une feuille supérieure (2) perméable aux liquides ;
une feuille arrière (3) imperméable aux liquides ; et
une partie centrale absorbante (4) comprenant un matériau absorbant (5) en son sein et étant prise en sandwich entre ladite feuille supérieure (2) et ladite feuille arrière (3) ;
dans lequel l'insert (1) comprend un périmètre formé par des premier et second bords transversaux (6, 7) et des premier et second bords longitudinaux (8, 9) reliant les premier et second bords transversaux (6, 7) ; une ligne médiane longitudinale (y) s'étendant sensiblement parallèlement auxdits premier et second bords longitudinaux (8, 9) et interposée entre eux de manière à diviser ledit insert (1) en une première moitié longitudinale entre ladite ligne médiane longitudinale (y) et ledit premier bord longitudinal (8) et une seconde moitié longitudinale entre ladite ligne médiane longitudinale (y) et ledit second bord longitudinal (9) ; et une ligne médiane transversale (x) s'étendant sensiblement parallèlement auxdits premier et second bords transversaux (6, 7) et interposée entre eux de manière à diviser ledit insert (1) en une première moitié transversale entre ladite ligne médiane transversale (x) et ledit premier bord transversal (6) et une seconde moitié transversale entre ladite ligne médiane transversale (x) et ledit second bord transversal (7) ;
**caractérisé en ce que** l'insert (1) comprend des plis ($F_o$, $F_o'$) s'étendant longitudinalement à proximité de chacun desdits premier et second bords longitudinaux (8, 9), dans lequel lesdits plis ($F_o$, $F_o'$) comprennent une surface de contact où une première partie de la feuille arrière (3) est en contact avec une seconde partie de la feuille arrière (3) et **en ce que** ladite surface de contact est une surface de ladite feuille arrière (3) tournée vers le vêtement.

2. Insert (1) selon la revendication 1 dans lequel ledit insert comprend des rebords ($F_L$, $F_L'$) s'étendant longitudinalement disposés de manière opposée le long des premier et second bords longitudinaux (8, 9) et dans lequel lesdits rebords ($F_L$, $F_L'$) sont pliés pour former les plis ($F_o$, $F_o'$) s'étendant longitudinalement, de préférence dans lequel les rebords ($F_L$, $F_L'$) s'étendant longitudinalement sont sensiblement exempts de matériau absorbant.

3. Insert (1) selon la revendication 2 dans lequel les rebords ($F_L$, $F_L'$) s'étendant longitudinalement comprennent la feuille supérieure (2) et la feuille arrière (3) reliées directement ou indirectement l'une à l'autre.

4. Insert (1) selon l'une quelconque des revendications précédentes comprenant au moins une paire de revers latéraux ($C_F$, $C_F'$) dans lequel au moins un desdits revers est positionné le long d'au moins une partie du premier bord longitudinal (8) et au moins un desdits revers est positionné le long d'au moins une partie du second bord longitudinal (9), de préférence dans lequel les revers comprennent un ou plusieurs élastiques (e, e') proximaux à un sommet de ceux-ci de telle sorte qu'une tension de ceux-ci amène les revers à faire saillie vers le haut en s'éloignant de la feuille supérieure (2).

**5.** Insert (1) selon la revendication 4 dans lequel les revers sont positionnés sensiblement à l'intérieur de rebords ($F_L$, $F_L$') s'étendant longitudinalement.

**6.** Insert (1) selon l'une quelconque des revendications précédentes dans lequel la partie centrale absorbante (4) comprend une enveloppe de partie centrale comprenant une couche supérieure (14) et une couche inférieure (15) et dans lequel le matériau absorbant (5) est entouré par ladite enveloppe de partie centrale entre lesdites couches supérieure et inférieure (14, 15) de l'enveloppe de partie centrale.

**7.** Insert (1) selon la revendication 6 dans lequel la partie centrale absorbante (4) comprend une ou plusieurs zones de fixation dans lesquelles la couche supérieure (14) de l'enveloppe de partie centrale adhère à la couche inférieure (15) de l'enveloppe de partie centrale de telle sorte qu'un ou plusieurs canaux (13) sensiblement exempts de matériau absorbant sont formés, de préférence à l'intérieur d'un périmètre de ladite partie centrale (4) de telle sorte que lesdits canaux (13) ne s'étendent pas jusque ni n'atteignent ledit périmètre.

**8.** Ensemble absorbant comprenant :

un insert (1) selon l'une quelconque des revendications 1 à 7 ; et
une enveloppe extérieure réutilisable (20).

**9.** Ensemble absorbant selon la revendication 8 dans lequel la enveloppe extérieure réutilisable (20) comprend :

des régions de taille avant (F) et arrière (B) présentant des bords le plus haut et le plus bas ; et
une région d'entrejambe (C) interposée entre les régions de taille avant (F) et arrière (B), dans lequel les bords les plus hauts de la région de taille forment une ouverture de taille et les bords les plus bas de la région de taille conjointement avec les extrémités latérales de la région d'entrejambe forment deux ouvertures pour les jambes disposées de manière opposée ;
et dans lequel au moins la surface de la région d'entrejambe (C) faisant face au corps est conçue pour recevoir un insert absorbant (1) selon l'une quelconque des revendications précédentes.

**10.** Ensemble absorbant selon la revendication 9 comprenant des coutures latérales refermables ou permanentes reliant les régions de taille avant et arrière, ladite enveloppe extérieure (20) se présentant de préférence sous la forme d'un sous-vêtement.

**11.** Kit de parties comprenant :

une pluralité d'inserts (1) selon l'une quelconque des revendications 1 à 7 ; et
une ou plusieurs enveloppes extérieures (20) selon l'une quelconque des revendications 8 à 10.

**12.** Kit selon la revendication 11 dans lequel la pluralité d'inserts (1) différent en au moins l'un des éléments suivants : taille, pouvoir absorbant, contenu biodégradable et/ou compostable, forme et nombre de composants contenus dans ceux-ci.

**13.** Insert (1) selon l'une quelconque des revendications précédentes comprenant en outre une couche de distribution d'acquisition (ADL) positionnée entre la feuille supérieure (2) et la partie centrale absorbante (4).

**14.** Insert (1) selon l'une quelconque des revendications précédentes dans lequel la feuille supérieure (2) est un non-tissé comprenant des fibres végétales, dans lequel lesdites fibres végétales comprennent, consistent de préférence en, des fibres récoltées autres que la pâte de bois, de telle sorte que ladite feuille supérieure (2) présente une teneur d'origine biologique allant d'environ 10 % à environ 100 % selon la norme ASTM D6866-10, procédé B, et facultativement, dans lequel ledit non-tissé comprend en outre des fibres synthétiques généralement en une quantité permettant d'offrir une intégrité structurale supplémentaire, telle que de 10 % à 60 % en poids dudit non-tissé.

**15.** Insert (1) selon l'une quelconque des revendications précédentes dans lequel la feuille arrière (3) comprend du bio-PE, et dans lequel le matériau absorbant comprend des particules polymères superabsorbantes, de préférence sélectionnées parmi le Bio-SAP à faible AUL présentant une AUL inférieure à 15 g/g et le Bio-SAP à haute AUL présentant une AUL supérieure à 15 g/g, telle que mesurée selon la procédé d'essai de la description.

**16.** Insert (1) selon l'une quelconque des revendications précédentes dans lequel au moins 85 %, de préférence au moins 90 % en poids, plus préférentiellement au moins 95 % en poids, en poids total de l'insert absorbant jetable (1) comprend des composants présentant une teneur d'origine biologique comprise d'environ 10 % à environ 100 %, de préférence d'environ 15 % à environ 100 %, encore plus préférentiellement d'environ 20 % à environ 100 %, selon la norme ASTM D6866-10, procédé B.

**17.** Procédé de fabrication d'inserts absorbants jetables, de préférence selon l'une quelconque des revendications précédentes, ledit procédé comprenant les étapes de :

(i) fourniture d'une première bande de matériau, de préférence un non-tissé perméable aux liquides ;

(ii) dépôt d'un matériau absorbant sur ladite première bande ;

(iii) fourniture d'une seconde bande de matériau, de préférence un non-tissé perméable aux liquides, et application de ladite seconde bande de matériau sur le matériau absorbant déposé, ou pliage de ladite première bande pour enfermer le matériau absorbant déposé dans celle-ci ;

(iv) assemblage des première et seconde bandes, ou la première bande pliée, au niveau des une ou plusieurs zones de fixation pour former une partie centrale absorbante comprenant un matériau absorbant entouré par ladite ou lesdites bande(s) ;

(v) application facultative d'une couche de distribution d'acquisition ;

(vi) prise en sandwich de la partie centrale absorbante, et de préférence de la couche de distribution d'acquisition, entre une couche perméable aux liquides et une couche sensiblement imperméable aux liquides disposée à l'opposé de celle-ci, de préférence la couche de distribution d'acquisition étant en contact avec la couche perméable aux liquides, pour former un insert absorbant jetable ;

**caractérisé en ce que** après l'étape (vi), une première étape de pliage est appliquée, dans lequel les premier et second bords longitudinaux dudit insert sont rentrés en pliant lesdits bords autour d'un axe de pliage s'étendant parallèlement à un sens machine de telle sorte que des plis ($F_o$, $F_o'$) s'étendant longitudinalement proximaux à chacun desdits premier et second bords longitudinaux (8, 9) sont formés, dans lequel lesdits plis ($F_o$, $F_o'$) comprennent une surface de contact où une première partie de la couche sensiblement imperméable aux liquides formant une feuille arrière (3) est en contact avec une seconde partie de ladite feuille arrière (3) et **en ce que** ladite surface de contact est une surface de ladite feuille arrière (3) tournée vers le vêtement.

18. Procédé selon la revendication 16 dans lequel après la première étape de pliage, une seconde étape de pliage est appliquée, dans lequel l'insert est plié autour d'une ligne médiane transversale (x) de telle sorte que les plis ($F_o$, $F_o'$) s'étendant longitudinalement renferment sensiblement d'autres parties de l'insert plié dans ceux-ci.

FIG. 1

FIG. 2

FIG. 3

FIG. 4

FIG. 5

FIG. 6

FIG. 7

## REFERENCES CITED IN THE DESCRIPTION

**Patent documents cited in the description**

- EP 3842017 A1
- EP 3659563 A1
- EP 3895673 A1
- US 2014257231 A1
- US 2021361498 A1
- US 20200054782 A1
- US 4414398 A
- EP 21152698
- WO 2008132630 A